# FASCICULE DE BREVET EUROPEEN

(11) **EP 3 206 714 B1**
(45) Date de publication et mention de la délivrance du brevet: **10.04.2019**
(21) Numéro de dépôt: 15771190.4
(22) Date de dépôt: 07.09.2015
(51) Int. Cl.: A61K 45/06, A61K 31/13, A61K 31/21, A61K 31/445, A61K 33/00, A61P 25/14, A61P 25/16, A61P 25/18, A61P 25/24, A61P 25/30

(54) **MÉDICAMENT POUR TRAITER UNE MALADIE LIÉE À UN DYSFONCTIONNEMENT DE LA TRANSMISSION SYNAPTIQUE DOPAMINERGIQUE**
ARZNEIMITTEL ZUR BEHANDLUNG EINER KRANKHEIT IM ZUSAMMENHANG MIT EINER DYSFUNKTION DER DOPAMINERGEN SYNAPTISCHEN ÜBERTRAGUNG
MEDICAMENT FOR TREATING A DISEASE RELATING TO A DYSFUNCTION OF THE DOPAMINERGIC SYNAPTIC TRANSMISSION

(30) Priorité: 17.10.2014 FR 1459982
(43) Date de publication de la demande: 23.08.2017
(73) Titulaire: L'Air Liquide Société Anonyme pour l'Etude et l'Exploitation des Procédés Georges Claude, 75007 Paris (FR); L'Institut du Cerveau et de la Moelle Epiniere, 75013 Paris (FR)
(72) Inventeur: MICHEL, Patrick, 75012 Paris (FR); LAVAUR, Jérémie, F-75007 Paris (FR); HIRSCH, Etienne, F-78000 Versailles (FR); LEMAIRE, Marc, F-75014 Paris (FR)
(74) Mandataire: Pittis, Olivier
(86) Numéro de dépôt international: PCT/FR2015/052371
(87) Numéro de publication internationale: WO 2016/059307

(56) Documents cités:
- WO-A1-2015/004347
- WO-A1-2015/004371
- CN-A- 101 147 741
- FR-A1- 2 858 233
- FR-A1- 2 952 305
- DAVID ET AL: "Nitrous Oxide and Xenon Prevent Amphetamine-Induced Carrier-Mediated Dopamine Release in a Memantine-Like Fashion and Protect Against Behavioral Sensitization", BIOLOGICAL PSYCHIATRY, ELSEVIER SCIENCE, NEW YORK, NY; US, vol. 60, no. 1, juillet 2006 (2006-07), pages 49-57, XP005512613, ISSN: 0006-3223, DOI: 10.1016/J.BIOPSYCH.2005.10.007

## Description

L'invention porte sur l'utilisation de xénon gazeux en tant que médicament inhalable, utilisé en combinaison avec un antagoniste des récepteurs N-Methyl-D-Aspartate (NMDA) au glutamate pour traiter, c'est-à-dire pour soigner, ralentir l'évolution, atténuer ou minimiser, une maladie du système nerveux central résultant d'un dysfonctionnement de la transmission synaptique dopaminergique résultant d'une attrition du corps cellulaire, d'une diminution de l'arborisation neuritique ou d'une réduction du fonctionnement synaptique chez un patient humain.

Les récepteurs/canaux NMDA sont des entités moléculaires de la membrane plasmique des cellules neuronales. Ces récepteurs, sont la cible des molécules de glutamate libérées dans l'espace synaptique et extra-synaptique, le glutamate étant un neurotransmetteur excitateur assurant la communication d'une cellule nerveuse à une autre.

La transmission dopaminergique est altérée dans un certain nombre de pathologies ou conditions pathologiques du système nerveux central, en particulier la maladie de Parkinson, les dyskinésies, la schizophrénie, le syndrome des jambes sans repos, le syndrome de Gilles de la Tourette, les addictions, les dépressions majeures et les troubles du déficit de l'attention, avec ou sans hyperactivité.

La mémantine, la nitromémantine, l'amantadine et l'ifenprodil sont des composés ayant une action antagoniste vis-à-vis du récepteur NMDA porté par les neurones, mais ces molécules sont aussi capables de produire un effet neurotrophique via un mécanisme impliquant les cellules gliales de type astrocytaire (Toyomoto et al, Neurosci Letters, 2005 ; Wu et al, Neuropsychopharmacology, 2009; Ossola et al, Neuropharmacology, 2009). Il en résulte alors une amélioration de la transmission nerveuse entre cellules neuronales.

Cependant, cet effet positif des antagonistes des récepteurs NMDA, en particulier de la mémantine, est limité car ces molécules ne sont pas dénuées d'effets indésirables, tel que confusion, vertiges, somnolence, céphalées, insomnies, agitation, hallucinations, vomissements, anxiété... qui viennent contrebalancer leur effet positif.

Le problème est donc de proposer un médicament permettant de traiter, de ralentir ou de minimiser une maladie du système nerveux central engendrée par, ou résultant d'un dysfonctionnement de la transmission synaptique dopaminergique dans le cas de certaines pathologies ou conditions pathologiques, notamment de la maladie de Parkinson, de dyskinésies, de la schizophrénie, du syndrome des jambes sans repos, du syndrome de Gilles de la Tourette, d'une dépression majeure et de troubles du déficit de l'attention, avec ou sans hyperactivité (Oades, Prog Brain Res, 2008; Beaulieu and Gainetdinov, Pharmacol Rev, 2011 ; Michel et al, Faseb J, 2013; Ferini-Strambi and Marelli, Expert Opin Pharmacother, 2014), tout en limitant les effets indésirables susmentionnés liés à l'utilisation de la mémantine.

Le document FR 2858233 divulgue des combinaisons de xénon gazeux et d'au moins un antagoniste des récepteurs NMDA gazeux (protoxyde d'azote) pour le traitement de comportements addictifs.

La solution selon la présente invention est un médicament contenant du xénon gazeux pour une utilisation en association avec au moins un antagoniste des récepteurs NMDA sous forme liquide ou solide, pour traiter une maladie du système nerveux central résultant d'un dysfonctionnement de la transmission synaptique dopaminergique résultant d'une attrition du corps cellulaire, d'une diminution de l'arborisation neuritique ou d'une réduction du fonctionnement synaptique chez un patient humain.

Dans le cadre de l'invention, le terme « traiter » est entendu au sens large et englobe non seulement « soigner » mais aussi « ralentir l'évolution », « atténuer » ou « minimiser » la maladie. Le traitement est donc total ou partiel.

Dit autrement, l'invention concerne une combinaison médicamenteuse comprenant du xénon gazeux et au moins un antagoniste des récepteurs NMDA sous forme liquide ou solide pour traiter une maladie causée par un dysfonctionnement de la transmission synaptique dopaminergique résultant d'une attrition du corps cellulaire, d'une diminution de l'arborisation neuritique ou d'une réduction du fonctionnement synaptique chez un patient humain.

En effet, dans le cadre de la présente invention, il a été mis en évidence que l'association de xénon et d'un antagoniste des récepteurs NMDA, telle la mémantine, conduit à une action synergique de ces composés et qu'une telle association peut constituer un traitement prometteur des maladies du système nerveux central résultant d'un dysfonctionnement de la transmission synaptique dopaminergique, en particulier de la maladie de Parkinson, des dyskinésies, de la schizophrénie, du syndrome des jambes sans repos, du syndrome de Gilles de la Tourette, d'une dépression majeure et des troubles du déficit de l'attention avec ou sans hyperactivité.

Une telle association repose notamment sur les modes d'action de ces composés.

Ainsi, le xénon possède des propriétés inhibitrices des voies de signalisation glutamatergiques excitatrices (Dinse et al, Br J Anaesth, 2005), via son action antagoniste sur les récepteurs NMDA, mais aussi sur les récepteurs α-amino-3-hydroxy-5-méthylisoazol-4-propionate (AMPA), ainsi que sur les récepteurs kainate, qui composent les récepteurs ionotropiques au glutamate.

Dès lors, l'association de xénon avec un composé antagoniste des récepteurs NMDA, en particulier la mémantine, la nitromémantine, l'amantadine et l'ifenprodil, conduit à une synergie d'action, sans risque d'augmenter les effets indésirables de l'antagoniste des récepteurs NMDA car une telle association permet d'utiliser des doses plus faibles de l'antagoniste utilisé.

En d'autres termes, le xénon permet de renforcer les effets bénéfiques de l'antagoniste des récepteurs NMDA, en particulier de la mémantine, du fait d'un effet synergique mais sans engendrer d'effets indésirables, tel que confusion, vertiges, somnolence, céphalées, insomnies, agitation, hallucinations, vomissements, anxiété...

De façon générale, une telle association permet de rétablir la transmission synaptique de neurones dopaminergiques « malades » dont le fonctionnement est altéré.

Selon le cas, le médicament gazeux ou combinaison médicamenteuse selon l'invention peut comprendre l'une ou plusieurs des caractéristiques suivantes :
- le xénon est sous forme administrable par inhalation, c'est-à-dire qu'il est destiné et apte à être inhalé par le patient.
- l'antagoniste des récepteurs NMDA est sous forme solide, en particulier sous forme de comprimé ou de gélule.
- l'antagoniste des récepteurs NMDA est choisi parmi la mémantine, la nitromémantine, l'amantadine et l'ifenprodil, ou tout autre composé analogue.
- la maladie causée par un dysfonctionnement de la transmission synaptique dopaminergique est choisie parmi la maladie de Parkinson, les dyskinésies, la schizophrénie, le syndrome des jambes sans repos, le syndrome de Gilles de la Tourette, les comportements addictifs, la dépression sévère et les troubles du déficit de l'attention avec ou sans hyperactivité.
- la maladie résulte de, ou est causée par un dysfonctionnement de la transmission synaptique dopaminergique engendrée par, ou résultant d'une attrition du corps cellulaire, d'une diminution de l'arborisation neuritique ou d'une réduction du fonctionnement synaptique.
- la dose journalière d'antagoniste des récepteurs NMDA administrée au patient est inférieure ou égale à 20 mg/jour.
- le xénon gazeux est administré au patient avant, concomitamment ou après administration de l'antagoniste des récepteurs NMDA, de préférence après administration de l'antagoniste des récepteurs NMDA.
- le xénon est mélangé à de l'oxygène, de préférence à au moins 21% en volume d'oxygène.
- le patient est un homme ou une femme, qu'il s'agisse d'un adulte ou d'un enfant.
- le médicament contient une quantité efficace de xénon.
- le médicament contient une quantité non anesthésique de xénon, i.e. sub-anesthésique.
- la proportion de xénon est comprise entre 10 et 80% en volume.
- la proportion de xénon est comprise entre 15 et 80% en volume.
- la proportion de xénon est d'au moins 20% en volume.
- la proportion de xénon est d'au plus 75% en volume.
- la proportion de xénon est d'au plus 60% en volume.
- la proportion de xénon est comprise entre 20 et 50% en volume.
- la proportion de xénon est comprise entre 20 et 40% en volume.
- le xénon est mélangé avec de l'oxygène juste avant ou au moment de son inhalation par le patient ou se présente sous forme d'un mélange gazeux « prêt à l'emploi » en pré-mélange avec de l'oxygène, et contient éventuellement un autre composé gazeux, par exemple de l'azote.
- on utilise un mélange constitué de xénon et d'oxygène.
- on utilise un mélange constitué de xénon, d'azote et d'oxygène.
- le mélange gazeux est administré au patient, une ou plusieurs fois par jour.
- le mélange gazeux est administré au patient pendant une durée d'inhalation de quelques minutes à quelques heures, typiquement entre 15 minutes et 6 heures, préférentiellement moins de 4 heures.
- la durée, la posologie et la fréquence d'administration sont fonction de l'évolution de l'état neurologique du patient considéré et seront préférentiellement fixées par le médecin ou personnel soignant en fonction de l'état neurologique du patient considéré.
- le xénon ou le mélange gazeux est conditionné dans une bouteille de gaz ayant une contenance (équivalent en eau) allant jusqu'à 50 litres, typiquement de l'ordre de 0,5 à 15 litres et/ou à une pression inférieure ou égale à 350 bar absolus, typiquement une pression comprise entre 2 et 300 bar.
- le xénon ou le mélange gazeux est conditionné dans une bouteille de gaz équipée d'un robinet ou d'un robinet à détendeur intégré permettant de contrôler le débit et éventuellement la pression du gaz délivré.
- le xénon ou le mélange gazeux est conditionné dans une bouteille de gaz en acier, en aluminium ou en matériaux composites.
- lors du traitement, l'administration au patient du xénon ou du mélange gazeux à base de xénon se fait par inhalation au moyen d'un masque facial ou nasal, de lunettes nasales ou au travers de tout autre système d'administration d'un gaz inhalable.

L'invention porte aussi sur une utilisation de xénon gazeux et d'au moins un antagoniste des récepteurs NMDA sous forme liquide ou solide pour fabriquer un médicament utilisable pour traiter une maladie causée par un dysfonctionnement de la transmission synaptique dopaminergique résultant d'une attrition du corps cellulaire, d'une diminution de l'arborisation neuritique ou d'une réduction du fonctionnement synaptique chez un patient humain.

Selon le cas, l'utilisation selon l'invention peut comprendre l'une ou plusieurs des caractéristiques techniques suivantes :
- le médicament gazeux ou la combinaison médicamenteuse comprend l'une ou plusieurs des caractéristiques décrites ci-avant.
- l'antagoniste des récepteurs NMDA est choisi parmi la mémantine, la nitromémantine, l'amantadine et l'ifenprodil.
- la maladie causée par un dysfonctionnement de la transmission synaptique dopaminergique résultant d'une attrition du corps cellulaire, d'une diminution de l'arborisation neuritique ou d'une réduction du fonctionnement synaptique est choisie parmi la maladie de Parkinson, les dyskinésies, la schizophrénie, le syndrome des jambes sans repos, le syndrome de Gilles de la Tourette, les comportements addictifs, la dépression sévère et les troubles du déficit de l'attention avec ou sans hyperactivité.

Selon un autre aspect, l'invention porte également sur une méthode de traitement thérapeutique pour traiter ou pour ralentir une maladie du système nerveux central résultant d'un dysfonctionnement de la transmission synaptique dopaminergique, méthode dans laquelle :
i) on identifie un patient humain souffrant d'une maladie du système nerveux central résultant d'un dysfonctionnement de la transmission synaptique dopaminergique résultant d'une attrition du corps cellulaire, d'une diminution de l'arborisation neuritique ou d'une réduction du fonctionnement synaptique,
ii) on administre par inhalation, audit patient, un médicament gazeux contenant du xénon, et
iii) on administre, audit patient, au moins un antagoniste des récepteurs NMDA sous forme liquide ou solide, de manière à traiter ladite maladie du système nerveux central.

Selon cette méthode de traitement thérapeutique, l'association de xénon et de l'antagoniste des récepteurs NMDA conduit à une action combinée de ces composés permettant une restauration du fonctionnement synaptique normal conduisant ainsi à un traitement, notamment au moins à un ralentissement de la maladie.

En particulier, une telle maladie est choisie parmi la maladie de Parkinson, les dyskinésies, la schizophrénie, le syndrome des jambes sans repos, le syndrome de Gilles de la Tourette, l'addiction, la dépression majeure ou les troubles du déficit de l'attention avec ou sans hyperactivité chez ledit patient.

De préférence, à l'étape i) :
- le patient humain est un homme ou une femme, qu'il s'agisse d'un adulte ou d'un enfant.
- l'identification du patient se fait par un médecin ou analogue.
- l'identification du patient se fait par examen technique de dépistage.
- l'anomalie de fonctionnement synaptique des neurones dopaminergiques susceptible d'engendrer un dysfonctionnement neurologique.

De préférence, à l'étape ii) :
- on administre, audit patient, au moins un antagoniste des récepteurs NMDA sous forme solide.
- on administre au moins un antagoniste des récepteurs NMDA, de préférence par voie entérale, i.e. la voie orale.
- on administre, audit patient, au moins un antagoniste des récepteurs NMDA sous forme de comprimé ou de gélule.
- on administre au patient de la mémantine ou un composé dérivé de la mémantine en tant qu'antagoniste des récepteurs NMDA.
- on administre au patient de la nitromémantine en tant qu'antagoniste des récepteurs NMDA.
- on administre au patient l'amantadine ou l'ifenprodil en tant qu'antagoniste des récepteurs NMDA.
- on administre au patient une dose journalière d'antagoniste des récepteurs NMDA inférieure ou égale à 20 mg/jour.
- on administre, audit patient, au moins un antagoniste des récepteurs NMDA avant, concomitamment, ou après inhalation de xénon par le patient.

De préférence, à l'étape iii) :
- la durée, la posologie et la fréquence d'administration du xénon sont choisies et/ou fixées en fonction de l'évolution de l'état neurologique du patient considéré.
- on administre une quantité efficace de xénon.
- on administre une quantité non anesthésique de xénon.
- on administre de 10 à 75% en volume de xénon, de préférence entre 20 et 50% en volume de xénon.
- on mélange le xénon avec de l'oxygène avant ou au moment de son inhalation par le patient, de préférence avec au moins 21% en volume d'oxygène.
- on administre un mélange gazeux prêt à l'emploi constitué de xénon et d'oxygène (mélange binaire).
- on administre un mélange gazeux prêt à l'emploi constitué de xénon, d'oxygène et d'azote (mélange ternaire).
- on administre le xénon gazeux une ou plusieurs fois par jour au patient.
- on administre le xénon gazeux au patient pendant une durée d'inhalation de quelques minutes à quelques heures, typiquement entre 15 minutes et 6 heures, préférentiellement moins de 4 heures.
- on administre le xénon gazeux au moyen d'un masque facial ou nasal, de lunettes nasales ou au travers de tout autre système ou dispositif d'administration de gaz à un patient.

### Exemples

Afin de démontrer l'effet synergique de la combinaison du xénon et d'un antagoniste des récepteurs NMDA selon la présente invention, un modèle cellulaire a été mis en place dans lequel le dysfonctionnement de la transmission synaptique dopaminergique est spontané.

Ce dysfonctionnement se caractérise par une attrition du corps cellulaire, une diminution de l'arborisation neuritique ainsi que par une réduction du fonctionnement synaptique, dans les conditions de culture utilisées (Wu et al, Neuropsychopharmacology, 2009).

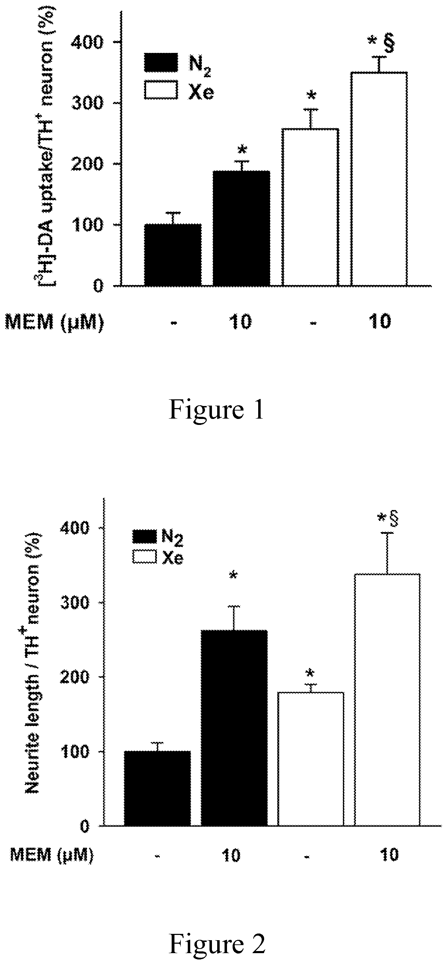

La technique mise en oeuvre est décrite ci-dessous et les résultats obtenus sont donnés dans les Tableaux 1 et 2 et illustrés par les Figure 1 et 2 ci-annexées montrant les effets synergiques du xénon associé à la mémantine dans un modèle cellulaire mimant une attrition neuronale en rapport avec un dysfonctionnement de la transmission synaptique dopaminergique.

### Protocole d'obtention des cultures primaires de mésencéphale

Des cultures sont préparées à partir de mésencéphale d'embryons de rats, prélevés sur des rates Wistar, au jour 15,5 de gestation.

Le procédé d'obtention des cultures de mésencéphale comprend l'obtention d'une suspension cellulaire homogène par dissociation mécanique, c'est-à-dire non enzymatique, du tissu embryonnaire, en utilisant du milieu L15 de Leibovitz (Sigma Aldrich).

Des aliquotes de cette suspension sont ajoutés à des plaques Nunc de 48 puits, qui ont été préalablement revêtues d'une fine couche de polyéthylènimine (1 mg/ml, tampon borate pH 8,3) pour permettre l'adhésion des cellules neuronales (Toulorge et coll., Faseb J, 2011).

La densité d'ensemencement est comprise entre environ 80 000 et 100 000 cellules/cm².

Les cultures de mésencéphale sont maintenues dans du milieu de culture Minimum Essential Medium (MEM), contenant 1g/l de glucose, 2 mM de L-glutamine, 1mM de pyruvate de sodium, des acides aminés non-essentiels et un cocktail pénicilline/streptomycine. La première semaine de culture, ce milieu est supplémenté par 10% de sérum foetal de veau et 10 % de sérum de cheval. A partir de la deuxième semaine, les concentrations en sérum sont réduites à 2 % (Gao et al, J Neurosci, 2002).

Jusqu'au moment où l'on évalue les effets des gaz d'intérêt, les cultures sont placées dans une enceinte conventionnelle thermostatée à 37°C, dans laquelle le CO₂ est maintenu à 5% en volume et où l'atmosphère est saturée en eau.

Le processus de dysfonctionnement des neurones dopaminergiques qui se met en place spontanément, résulte de l'attrition neuronale et de la réduction de l'arborisation neuritique.

### Traitements pharmacologiques des cultures

Un bloqueur des récepteurs NMDA, à savoir la mémantine, est ajouté aux cultures juste avant la mise sous atmosphère gazeuse contrôlée et le traitement est prolongé jusqu'à la fixation des cultures.

### Maintenance des cultures sous atmosphère gazeuse contrôlée

Une fois les traitements pharmacologiques effectués, les boîtes multi-puits contenant les cellules en culture et la boite servant à l'humidification du compartiment interne de la chambre, sont placées sur une embase métallique qui reçoit la chambre d'incubation en Plexiglas. L'embase et la chambre en Plexiglas sont réunies par vissage, de manière jointive.

On injecte ensuite un mélange gazeux d'intérêt comprenant (% en volume) : 20 % d'O₂, 5 % de CO₂ et 75 % de gaz test, dans la chambre d'incubation, avec valves d'entrée et de sortie ouvertes, tout en contrôlant le débit de sortie grâce à un débitmètre. Les gaz tests sont de l'azote (N₂) et du xénon (Xe).

Le débit de sortie de référence, fixé pour l'air à 10 litres/mn, est corrigé en fonction de la densité du mélange utilisé. Lorsque la mesure du CO₂, atteint 5 % en sortie, on stoppe l'injection du mélange gazeux et la chambre est rendue totalement étanche en fermant les valves d'entrée et de sortie. La chambre d'exposition est alors placée dans une enceinte à 37°C pendant les 7 jours du protocole expérimental.

### Immunodétection de la protéine tyrosine hydroxylase (TH) et comptages cellulaires

Après rupture de l'étanchéité par ouverture des valves d'entrée et de sortie et dévissage de la chambre de son embase, les cultures sont fixées avec 4% de formaldéhyde dans du PBS pendant 12 mn et ensuite incubées à 4°C avec un anticorps monoclonal anti-TH (ImmunoStar ; dilution 1:5000) pendant 2 jours, de manière à révéler la présence des neurones dopaminergiques.

La révélation de cet anticorps est obtenue avec un anticorps secondaire anti-souris couplé à de l'Alexa Fluor 555 (Life Technologies; dilution 1:300 dans du PBS).

L'acquisition des images et le comptage des neurones dopaminergiques (TH⁺) sont réalisés avec un imageur automatisé de type Arrayscan XTI et le logiciel d'analyse d'image HCS Studio (ThermoFischer Scientific). Cette analyse nous permet d'avoir une estimation du nombre de neurones TH⁺ / puits de culture.

### Mesure des neurites portés par les neurones dopaminergiques

Les cultures utilisées pour le comptage des neurones dopaminergiques servent aussi à la mesure de la longueur des neurites portés par les neurones TH⁺ dopaminergiques.

Ce paramètre est évalué avec le même logiciel HCS Studio.

### Mesure de recapture de dopamine tritiée

La mesure de recapture en dopamine est effectuée en utilisant de la dopamine tritiée (30-60 Ci/mmol, PerkinElmer), selon un protocole décrit précédemment par Toulorge et collègues (Faseb J, 2011). La mesure de recapture est effectuée dans des cultures produites en même temps et dans les mêmes conditions que celles utilisées pour les études de comptage cellulaire. Pour chaque puits de culture, le niveau de recapture en dopamine tritiée est rapporté à la valeur estimée moyenne du nombre de neurones dopaminergiques TH⁺ dans chaque condition de traitement.

Les résultats obtenus dans ce modèle cellulaire de dysfonctionnement de la transmission synaptique dopaminergique, sont résumés dans les Tableaux 1 et 2 suivants et sont représentés dans les Figures 1 et 2, annexées ci-après.

**Tableau 1 : Recapture de dopamine tritiée par neurone dopaminergique**

| Cultures de mésencéphale à J 14 | | |
|---|---|---|
| Traitements J 7 - J 14 (7 jours) | Mélange gazeux (20% O₂ + 5% CO₂ + 75 % gaz testé) ; % en volume | Recapt ure dopamine/neurone TH⁺ |
| Groupe témoin | N₂ | - |
| Groupe mémantine (10 µM) | N₂ | + |
| Groupe Xénon seul | Xe | ++ |
| Groupe Xénon + mémantine (10 µM) | Xe | +++ |

**Tableau 2 : Longueur des neurites / neurone dopaminergique**

| Cultures de mésencéphale à J 14 | | |
|---|---|---|
| Traitements J 7 - J 14 (7 jours) | Mélange gazeux (20% O₂ + 5% CO₂ + 75 % gaz testé) ; % en volume | neurites/neurone TH⁺ |
| Groupe témoin | N₂ | - |
| Groupe mémantine (10 µM) | N₂ | ++ |
| Groupe Xénon seul | Xe | + |
| Groupe Xénon + mémantine (10 µM) | Xe | +++ |

Dans les deux tableaux précédents, une réponse favorable, synonyme d'une élévation de la recapture de dopamine par neurone TH⁺ (Tableau 1) ou d'une augmentation de la longueur des neurites par neurone TH⁺ (Tableau 2), en présence des traitements d'intérêt, est désignée par un signe « + », « ++ », « +++ », selon l'importance de cette réponse.

A l'inverse, une absence de réponse est représentée par un signe « - ».

Au vu des résultats obtenus illustrés sur les Figures ci-annexées, on constate que la combinaison du xénon et de la mémantine qui est un antagoniste des récepteurs NMDA, conduit à un effet synergique significativement supérieur à celui produit par chaque molécule, prise séparément, que le paramètre mesuré soit la recapture en dopamine tritiée / neurone TH⁺ ou la longueur des neurites / neurone TH⁺.

La mémantine exerce un effet significatif dans ce modèle cellulaire et vis-à-vis des deux paramètres étudiés, lorsqu'elle est appliquée seule à 10 µM, sous atmosphère contenant 75 % d'azote. Le xénon possède aussi une activité également significative lorsqu'il remplace l'azote. De plus, le xénon potentialise les effets obtenus en présence de mémantine.

Les résultats illustrés sur les Figures 1 et 2 font apparaître les effets synergiques du xénon et de la mémantine dans le modèle cellulaire de dysfonction synaptique dopaminergique.

Ces résultats ont été obtenus sur des cultures de mésencéphale de rat qui ont été placées à partir du jour 7 de culture jusqu'au jour 14 sous atmosphère contenant 75 % d'azote (N₂ 75) ou 75 % de xénon (Xe 75), en présence ou non de mémantine (MEM), testée à 10 µM.

Sur la Figure 1, la recapture de dopamine tritiée, considérée comme un index de fonction synaptique et de différenciation des neurones dopaminergiques, est mesurée sur cultures vivantes à J14.

Le nombre de neurones TH⁺ dopaminergiques est estimé sur des cultures soeurs, qui sont produites à partir d'une même suspension cellulaire de départ et sont fixées à J14.

Les niveaux de recapture en dopamine par neurone TH⁺ sont exprimés en % (± SEM) de cultures non traitées, cultivées sous une atmosphère contenant 75 % d'azote (condition témoin).

Ainsi, l'étude statistique faite par une ANOVA (ANalysis Of VAriance) de Kruskal-Wallis par rangs, suivie d'un test de Student-Newman-Keuls (n= 9 pour chaque point expérimental) démontre que :
- le xénon seul lorsqu'il remplace l'azote, la mémantine seule dans l'azote et la mémantine dans le xénon produisent un effet significativement supérieur à celui observé dans la condition, azote seul (^{*} p<0.05, augmenté vs cultures témoins à J14 sous 75 % d'azote).
- le xénon lorsqu'il est combiné à la mémantine, engendre un effet synergique et conduit à un résultat significativement supérieur à celui observé dans les conditions, xénon seul ou mémantine sous azote (^{§} p<0.05, augmenté à J14 vs cultures exposées au xénon seul ou à de la mémantine dans une atmosphère contenant de l'azote).

Sur la Figure 2, la longueur des neurites par neurone TH⁺, une mesure du potentiel des neurones dopaminergiques à pouvoir former des connections synaptiques avec d'autres neurones, est réalisée sur des cultures fixées à J14.

Les résultats sont exprimés en % (± SEM) de cultures non traitées, maintenues dans une atmosphère témoin contenant 75 % d'azote (condition témoin).

Ainsi, l'étude statistique faite par une ANOVA (ANalysis Of VAriance) de Kruskal-Wallis par rangs, suivie d'un test de Student-Newman-Keuls (n= 9 pour chaque point expérimental) démontre que :
- le xénon seul lorsqu'il remplace l'azote, la mémantine seule dans l'azote et la mémantine dans le xénon exercent un effet significativement supérieur à celui observé dans la condition témoin, azote seul (^{*} p<0.05, augmenté vs cultures témoins à J14 sous 75 % d'azote).
- le xénon lorsqu'il est combiné à la mémantine, exerce un effet significativement supérieur à celui observé dans les conditions xénon seul ou mémantine sous azote (^{§} p<0.05, augmenté à J14 vs cultures exposées au xénon seul ou à de la mémantine dans une atmosphère contenant de l'azote).

Les essais ci-dessus ont été réalisés avec de la mémantine en tant qu'antagoniste des récepteurs NMDA. Toutefois, ces résultats similaires seraient obtenus avec des composés du même type, analogues ou similaires, tel que nitromémantine, l'amantadine ou l'ifenprodil.

Le xénon conduit donc, lorsqu'il est associé à un antagoniste des récepteurs NMDA, telle la mémantine, la nitromémantine, l'amantadine ou l'ifenprodil, à un effet synergique dans le traitement, notamment le ralentissement de l'évolution, de maladies liées à un dysfonctionnement de la transmission synaptique dopaminergique, comme la maladie de Parkinson, les dyskinésies, la schizophrénie, le syndrome des jambes sans repos, le syndrome de Gilles de la Tourette, l'addiction, la dépression lorsqu'elle est majeure et les troubles du déficit de l'attention avec ou sans hyperactivité.

## Revendications

1. Combinaison médicamenteuse comprenant du xénon gazeux et au moins un antagoniste des récepteurs NMDA sous forme liquide ou solide pour traiter une maladie causée par un dysfonctionnement de la transmission synaptique dopaminergique résultant d'une attrition du corps cellulaire, d'une diminution de l'arborisation neuritique ou d'une réduction du fonctionnement synaptique chez un patient humain, dans laquelle :
- l'antagoniste des récepteurs NMDA est choisi parmi la mémantine, la nitromémantine, l'amantadine et l'ifenprodil, et
- la maladie causée par un dysfonctionnement de la transmission synaptique dopaminergique est choisie parmi la maladie de Parkinson, les dyskinésies, la schizophrénie, le syndrome des jambes sans repos, le syndrome de Gilles de la Tourette, les comportements addictifs, la dépression sévère et les troubles du déficit de l'attention avec ou sans hyperactivité.

2. Combinaison médicamenteuse selon la revendication précédente, **caractérisée en ce que** la proportion de xénon est comprise entre 10 et 80% en volume.

3. Combinaison médicamenteuse selon l'une des revendications précédentes, **caractérisée en ce que** l'antagoniste des récepteurs NMDA est sous forme solide, en particulier sous forme de comprimé ou de gélule.

4. Combinaison médicamenteuse selon l'une des revendications précédentes, **caractérisée en ce que** le xénon est mélangé avec de l'oxygène.

5. Combinaison médicamenteuse selon la revendication 4, **caractérisée en ce que** le xénon est mélangé avec au moins 21% en volume d'oxygène.

6. Combinaison médicamenteuse selon l'une des revendications précédentes, **caractérisée en ce que** le xénon est mélangé avec de l'oxygène ou avec de l'oxygène et de l'azote.

7. Combinaison médicamenteuse selon l'une des revendications précédentes, **caractérisée en ce que** la proportion de xénon est d'au moins 20% en volume.

8. Combinaison médicamenteuse selon l'une des revendications précédentes, **caractérisée en ce que** la proportion de xénon est d'au plus 75% en volume.

9. Combinaison médicamenteuse selon l'une des revendications précédentes, **caractérisée en ce que** la proportion de xénon est d'au plus 60% en volume.

10. Utilisation de xénon gazeux et d'au moins un antagoniste des récepteurs NMDA sous forme liquide ou solide pour fabriquer un médicament pour traiter une maladie causée par un dysfonctionnement de la transmission synaptique dopaminergique résultant d'une attrition du corps cellulaire, d'une diminution de l'arborisation neuritique ou d'une réduction du fonctionnement synaptique chez un patient humain, dans quelle :
- l'antagoniste des récepteurs NMDA est choisi parmi la mémantine, la nitromémantine, l'amantadine et l'ifenprodil, et
- la maladie causée par un dysfonctionnement de la transmission synaptique dopaminergique est choisie parmi la maladie de Parkinson, les dyskinésies, la schizophrénie, le syndrome des jambes sans repos, le syndrome de Gilles de la Tourette, les comportements addictifs, la dépression sévère et les troubles du déficit de l'attention avec ou sans hyperactivité.

## Patentansprüche

1. Arzneimittelkombination, umfassend gasförmiges Xenon und mindestens einen NMDA-Rezeptorantagonisten in flüssiger oder fester Form zur Behandlung einer Krankheit, die durch eine Dysfunktion der dopaminergen synaptischen Übertragung verursacht wird, die sich aus Zellkörperabrieb, verminderter neuritischer Arborisierung oder verminderter synaptischer Funktion bei einem menschlichen Patienten ergibt, wobei:
- der NMDA-Rezeptorantagonist ausgewählt ist aus Memantin, Nitromemantin, Amantadin und Ifenprodil, und
- die durch eine Dysfunktion der dopaminergen synaptischen Übertragung verursachte Krankheit ausgewählt ist aus Parkinson, Dyskinesien, Schizophrenie, Restless Leg Syndrom, Tourette-Syndrom, Suchtverhalten, schwerer Depression und Aufmerksamkeitsstörung mit oder ohne Hyperaktivität.

2. Arzneimittelkombination nach dem vorstehenden Anspruch, **dadurch gekennzeichnet, dass** der Anteil von Xenon zwischen 10 und 80 Vol.-% beträgt.

3. Arzneimittelkombination nach einem der vorstehenden Ansprüche, **dadurch gekennzeichnet, dass** der NMDA-Rezeptorantagonist in fester Form vorliegt, insbesondere in Form von Tablette oder Gelkapsel.

4. Arzneimittelkombination nach einem der vorstehenden Ansprüche, **dadurch gekennzeichnet, dass** Xenon mit Sauerstoff vermischt ist.

5. Arzneimittelkombination nach Anspruch 4, **dadurch gekennzeichnet, dass** Xenon mit mindestens 21 Vol.-% Sauerstoff vermischt ist.

6. Arzneimittelkombination nach einem der vorstehenden Ansprüche, **dadurch gekennzeichnet, dass** Xenon mit Sauerstoff oder mit Sauerstoff und Stickstoff vermischt ist.

7. Arzneimittelkombination nach einem der vorstehenden Ansprüche, **dadurch gekennzeichnet, dass** der Anteil von Xenon mindestens 20 Vol.-% beträgt.

8. Arzneimittelkombination nach einem der vorstehenden Ansprüche, **dadurch gekennzeichnet, dass** der Anteil von Xenon höchstens 75 Vol.-% beträgt.

9. Arzneimittelkombination nach einem der vorstehenden Ansprüche, **dadurch gekennzeichnet, dass** der Anteil von Xenon höchstens 60 Vol.-% beträgt.

10. Verwendung von gasförmigem Xenon und mindestens einem NMDA-Rezeptorantagonisten in flüssiger oder fester Form zur Herstellung eines Medikaments zur Behandlung einer Krankheit, die durch eine Dysfunktion der dopaminergen synaptischen Übertragung verursacht wird, die sich aus Zellkörperabrieb, verminderter neuritischer Arborisierung oder verminderter synaptischer Funktion bei einem menschlichen Patienten ergibt, wobei:
- der NMDA-Rezeptorantagonist ausgewählt ist aus Memantin, Nitromemantin, Amantadin und Ifenprodil, und
- die durch eine Dysfunktion der dopaminergen synaptischen Übertragung verursachte Krankheit ausgewählt ist aus Parkinson, Dyskinesien, Schizophrenie, Restless Leg Syndrom, Tourette-Syndrom, Suchtverhalten, schwerer Depression und Aufmerksamkeitsstörung mit oder ohne Hyperaktivität.

## Claims

1. Drug combination comprising gaseous xenon and at least one antagonist of the NMDA receptors in a liquid or solid form for treating a disease caused by a dysfunction of the dopaminergic synaptic transmission resulting from an attrition of the cell body, a reduction of the neuritic arborisation or a reduction of synaptic functioning in a human patient, wherein:
- the antagonist of the NMDA receptors is selected from among memantine, nitromemantine, amantadine and ifenprodil, and
- the disease caused by a dysfunction of the dopaminergic synaptic transmission is selected from among Parkinson's disease, dyskinesia, schizophrenia, restless legs syndrome, Tourette's syndrome, addictive behaviours, severe depression and attention deficit disorders with or without hyperactivity.

2. Drug combination according to the preceding claim, **characterised in that** the proportion of Xenon ranges from 10 to 80% by volume.

3. Drug combination according to one of the preceding claims, **characterised in that** the antagonist of the NMDA receptors is in a solid form, in particular a tablet or a capsule.

4. Drug combination according to one of the preceding claims, **characterised in that** the xenon is mixed with oxygen.

5. Drug combination according to claim 4, **characterised in that** the xenon is mixed with at least 21% of oxygen by volume.

6. Drug combination according to one of the preceding claims, **characterised in that** the xenon is mixed with oxygen or with oxygen and nitrogen.

7. Drug combination according to one of the preceding claims, **characterised in that** the proportion of xenon is of at least 20% by volume.

8. Drug combination according to one of the preceding claims, **characterised in that** the proportion of xenon is of at most 75% by volume.

9. Drug combination according to one of the preceding claims, **characterised in that** the proportion of xenon is of at most 60% by volume.

10. Use of gaseous xenon and of at least one antagonist of the NMDA receptors in a liquid or solid form to produce a medicament for treating a disease caused by a dysfunction of the dopaminergic synaptic transmission resulting from an attrition of the cell body, a reduction of the neuritic arborisation or a reduction of synaptic functioning in a human patient, wherein:
- the antagonist of the NMDA receptors is selected from among memantine, nitromemantine, amantadine and ifenprodil, and
- the disease caused by a dysfunction of the dopaminergic synaptic transmission is selected from among Parkinson's disease, dyskinesia, schizophrenia, restless legs syndrome, Tourette's syndrome, addictive behaviours, severe depression and attention deficit disorders with or without hyperactivity.
